(19) Europäisches Patentamt
European Patent Office
Office européen des brevets

(11) Numéro de publication : **0 187 798**
**B1**

# (12) FASCICULE DE BREVET EUROPÉEN

(45) Date de publication du fascicule du brevet : **30.11.88**

(51) Int. Cl.[4] : **C 07 H 15/04**, C 07 H 15/207, C 07 H 15/10

(21) Numéro de dépôt : **85903310.2**

(22) Date de dépôt : **18.07.85**

(86) Numéro de dépôt international : **PCT/FR 85/00197**

(87) Numéro de publication internationale : **WO/8600906 (13.02.86 Gazette 86/04)**

## (54) PROCEDE DE SYNTHESE D'ALDOSIDES OU D'OLIGOALDOSIDES D'ALKYLE, DE CYCLOALKYLE OU D'ALCENYLE.

(30) Priorité : **19.07.84 FR 8411466**

(43) Date de publication de la demande : **23.07.86 Bulletin 86/30**

(45) Mention de la délivrance du brevet : **30.11.88 Bulletin 88/48**

(84) Etats contractants désignés : **AT BE CH DE FR GB IT LI LU NL SE**

(56) Documents cités :
**EP-A- 0 106 522**
**DE-A- 1 905 523**
**US-A- 2 390 507**
**Carbohydrate Research, tome 110, 1982, Elsevier Scientific Publishing Company, (Amsterdam, NL) J. Defaye et al.: "The behaviour of cellulose, amylose, and beta-D-xylan towards anhydrous hydrogen fluoride", pages 217-227, voir pages 217-219**
**"The Carbohydrates (Chemistry and Biochemistry)" Tome IA (1972), page 181**

(73) Titulaire : **BEGHIN-SAY SA**
**F-59239 Thumeries (FR)**

(72) Inventeur : **DEFAYE, Jacques**
**202, chemin du Vercors**
**F-38330 Saint Dismier (FR)**
Inventeur : **WONG, Emile**
**117, cours Jean Jaurès**
**F-38000 Grenoble (FR)**
Inventeur : **PEDERSEN, Christian**
**Bredesvinget 18**
**DK-2830 Virum (DK)**
Inventeur : **CHEDIN, Jean**
**1ter, rue Mornay**
**F-75004 Paris (FR)**
Inventeur : **BOUCHU, Alain**
**11, av. R. Rosselini**
**F-69100 Villeurbanne (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

EP 0 187 798 B1

Jouve, 18, rue St-Denis, 75001 Paris, France

## Description

L'invention a pour objet un procédé amélioré de synthèse d'aldosides ou de oligo-aldosides d'alkyle, de cycloalkyle ou d'alcényle.

Les aldosides ou polyaldosides d'alkyle sont des produits industriels bien connus qui peuvent être utilisés à des fins très diverses et, notamment comme produits intermédiaires pour l'obtention de surfactants ou dans la fabrication de mousses de polyuréthanes, par exemple.

Etat de la technique

Il est connu de synthétiser les aldosides ou oligo-aldosides d'alkyle en faisant réagir un composé hydroxylé et de l'amidon en présence d'un catalyseur acide :

Le brevet US 2276621 décrit la préparation de méthyl-D-glucopyranosides par alcoolyse de l'amidon en présence d'un catalyseur acide tel que l'acide sulfurique. Dans ce cas, la réaction est conduite à 100 °C pendant 2 heures en présence d'un excès de méthanol, afin d'augmenter le rendement en méthyl-glucosides.

Le brevet US 3346558 porte sur la préparation de glucosides de polyols selon un procédé en continu. Ainsi, les glucosides de l'éthylène glycol, du glycérol, du sorbitol sont obtenus par passage d'un mélange d'amidon, d'un polyalcool et d'un catalyseur acide tel que l'acide paratoluène sulfonique ou un acide de Lewis comme l'éthérate de trifluorure de bore dans une extrudeuse chauffée à une température supérieure à 170 °C.

Le brevet US 3375243 décrit la préparation de glucosides d'alkyle et plus particulièrement de méthyl-D-glucopyranosides à partir de glucose ou d'amidon en présence d'un excès de méthanol (3 moles par équivalent monosaccharidique) et d'un catalyseur acide tel que l'acide paratoluène sulfonique (0,0025 à 0,1 mole par équivalent monosaccharide). La réaction est conduite dans un réacteur en quelques minutes à une température comprise entre 100 et 250 °C sous une pression maximale de 15 à 20 bars. Dans ces conditions, le taux de conversion en méthyl-glucosides est de l'ordre de 85 à 90 %.

Dans le brevet US 4223129, la préparation de glucosides d'alkyle est réalisée selon un procédé en continu à partir d'amidon ou d'autres polyosides. L'amidon est mis en suspension dans un alcool en présence d'un catalyseur acide, minéral ou organique, est envoyé sous pression dans un serpentin possédant une zone de chauffage intense bien délimitée dans laquelle a lieu la réaction. Cet appareillage est inspiré des brevets US 2735792 et US 3617383. En particulier, dans le cas de la préparation de méthyl-D-glucopyranosides, la température de la réaction est de l'ordre de 160 à 180 °C, et la pression est régulée en fonction du temps de contact désiré dans la zone de chauffe (8 à 20 minutes). Le rapport méthanol/amidon peut varier de 15 : 1 à 6,5 : 1 alors que le taux de catalyseur est de l'ordre de 0,005 mole par équivalent monosaccharidique. Dans ces conditions, le rendement en méthyl-glucosides varie de 65 à 90 %, en mélange essentiellement avec des di-et tri-saccharides de réversion. Les auteurs indiquent que ce procédé est applicable à la préparation de glucosides d'alcool en $C_2$ à $C_{18}$. Complétant ce procédé, le brevet US 4329449 décrit une méthode de cristallisation de méthyl-alpha-D-glucopyranoside avec recyclage des eaux-mères.

Dans le même domaine, le brevet US 3839318 revendique la préparation de glucosides d'alkyle par réaction directe du glucose avec un alcool en présence d'acide sulfurique, en travaillant à chaud sous pression réduite. Les rendements en glucosides d'alkyle varient dans ces conditions de 25 à 60 %, le reste étant constitué d'oligosaccharides de réversion alkylés.

Un autre procédé en continu est décrit dans la demande de brevet EP 96917. La préparation de glucosides d'alkyle ($C_8$ à $C_{15}$) est conduite par réaction d'un aldose, aldoside ou polyaldoside avec un excès de 50 % d'alcool en présence d'un catalyseur acide en particulier l'acide paratoluène sulfonique, à une température comprise entre 80° et 150 °C. Dans ces conditions un apport continu de glucose est réalisé, l'eau issue de la réaction étant éliminée au fur et à mesure. D'autres méthodes proposent l'emploi de résines échangeuses d'ions acides.

Ainsi le brevet US 2606186 décrit la préparation de méthyl-glucoside par réaction du glucose avec le méthanol en présence d'une résine sulfonique acide. Le temps de réaction est de 1 à 48 h et la température de l'ordre de 100 °C pour éviter toute dégradation de la résine. Les méthyl-glucosides cristallisés après concentration du mélange sont séparés par centrifugation.

Une autre technique consiste en la préparation de glucosides d'alkyle à longue chaîne par trans-glucosylation.

Le brevet US 3219656 décrit la préparation de glucosides d'alkyle ($C_4$ à $C_{18}$) par trans-glucosylation en présence de résine sulfonique acide, il est montré que la méthode décrite par le brevet US 2606186 est seulement applicable à la préparation du méthyl-glucoside. Ainsi, la préparation de glucosides d'alkyle comportant plus de 4 carbones est réalisée par transglucosylation du butyl-glucoside, lui-même obtenu à partir du méthyl-alpha-D-glucopyranoside. La préparation de glucosides d'alkyle selon cette technique est décrite également dans le brevet DE 1905523 où l'acide sulfurique est utilisé comme catalyseur. L'emploi de l'acide paratoluène sulfonique pour la préparation de glucosides d'alkyle selon cette méthode est préconisé dans la demande de brevet EP 92875.

Le traitement à chaud en milieu basique d'un mélange brut de glucosides d'alkyle est décrit dans le brevet US 3450690, il permet d'éliminer les impuretés et de faciliter ainsi la cristallisation du méthyl glucoside. De même, le traitement d'un mélange brut de glucosides d'alkyle par une résine échangeuse d'ions basique permet de stabiliser la couleur du mélange (US 3565885).

Il est par ailleurs connu par la publication Carbohydr. Res., 110 (1982) 217, Defaye et al, de dissoudre des polysaccharides (hexosanes et pentosanes) comme la cellulose ou le xylane dans le fluorure d'hydrogène ce qui conduit à la formation d'oligosaccharides par fluorolyse.

## Objet de l'invention

L'objet de l'invention est de proposer un nouveau procédé industriel permettant d'obtenir, avec un bon rendement, des aldosides ou oligo-aldosides d'alkyle, de cycloalkyle ou d'alcényle.

Le procédé est caractérisé en ce que l'on fait réagir un alcool aliphatique ou cycloaliphatique saturé ou un alcool aliphatique ou cycloaliphatique comportant une double liaison éthylénique non située en alpha du groupement hydroxyle avec un aldose, un aldoside, ou un polyaldoside dans un solvant et réactif constitué par du fluorure d'hydrogène.

L'utilisation de fluorure d'hydrogène comme solvant et réactif permet l'accès aux alkyl-glycopyranosides à partir de polyosides tels que l'amidon.

En effet, en solution dans le fluorure d'hydrogène (HF), l'amidon solubilisé dans ce solvant se transforme principalement en fluorure de glucopyranosyle, ainsi que quelques composés secondaires provenant de l'auto-condensation du glucose (produits de réversion).

Le schéma ci-dessous résume le mécanisme présumé de la réaction qui fait l'objet de l'invention.

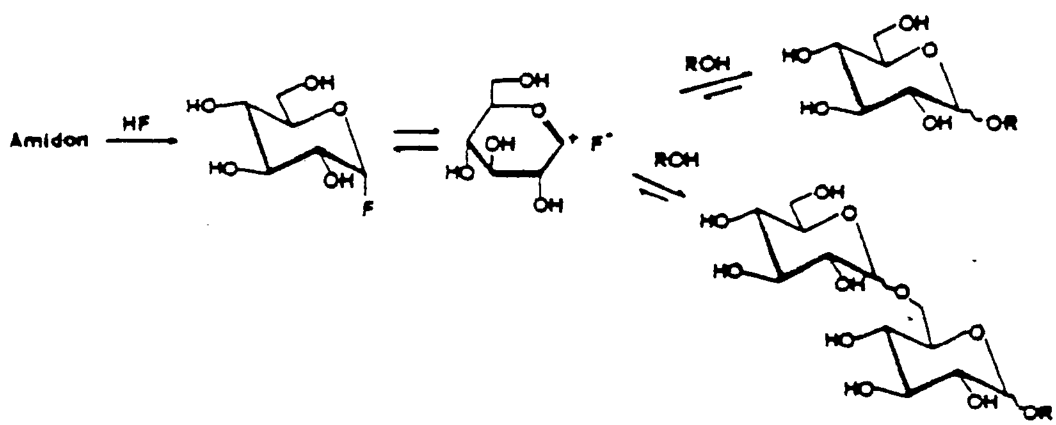


Un intermédiaire ion glycosyl-oxycarbénium, stabilisé par l'anion fluorure, en équilibre avec le fluorure de glycopyranosyle a été postulé dans cette réaction pour expliquer la rapidité de l'hydrolyse observée ainsi que la formation de produits de réversion lorsque l'équilibre est déplacé par concentration de la solution (Carbohydr. Res., 110 (1982) 217-227) (Schéma).

L'addition d'un alcool au milieu réactionnel conduit au glycoside correspondant, l'existence de deux anomères s'expliquant par l'ion oxycarbénium intermédiaire et leur proportion, par des facteurs thermodynamiques (Schéma). Des glycosides d'alkyle-disaccharidiques préférentiellement liés (1 —> 6) (Schéma), mais également (1 —> 2), (1 —> 3), et (1 —> 4) sont susceptibles d'être formés sous certaines conditions dans cette réaction. Quelques glycosides d'alkyle oligosaccharidiques très minoritaires peuvent également se former selon ce schéma réactionnel, dans les conditions décrites dans le présent brevet.

Le but fixé a donc été d'obtenir des alkyl-glycosides avec de bons rendements à partir d'oses et de polysaccharides utilisant ce schéma réactionnel.

La réaction est, de préférence, effectuée en milieu liquide. La température et la pression, contrairement à l'art antérieur ne sont pas des conditions déterminantes pour la réaction, elles conditionnent principalement la vitesse à laquelle la réaction est effectuée. Dans les cas favorables, on préférera opérer à température ambiante ou voisine de l'ambiante, et à pression atmosphérique car c'est finalement la solution la plus aisée. Cette température peu élevée permet de diminuer le pourcentage de sous-produits ainsi que la coloration et les problèmes qu'elle soulève. Mais la réaction peut se faire à une température inférieure (jusqu'à — 25 °C). Elle peut aussi être conduite à une température supérieure, dans les cas où cela est reconnu souhaitable, à condition que l'on utilise bien entendu les appareils appropriés résistant à la pression et la corrosion.

Par ailleurs ce procédé se traduit par une absence de produits de décomposition du type furannique résultant de déshydratation alors que ces produits sont constatés avec les acides minéraux et les résines acides.

D'autres avantages notés par rapport aux procédés antérieurs connus portent sur la possibilité de

recycler les réactifs, sur la formation préférentielle de l'anomère glucopyranosidique-alpha-D par rapport à l'anomère bêta-D, et enfin sur l'absence de produits de l'hydrolyse partielle des polysaccharides de départ.

Les alcools qui peuvent convenir sont nombreux et seront choisis selon le produit final que l'on désire obtenir.

A titre indicatif et non limitatif on peut citer les alcools aliphatiques ou cycloaliphatiques suivants :

- Les alcools primaires de formule R-OH où R est un groupe alkyle linéaire ou ramifié de $C_1$ à $C_{20}$ ou un groupe alkyle de $C_4$ à $C_{20}$ comportant au moins un groupe cycloalkyle, ou un groupe alkyle linéaire ou ramifié de $C_3$ à $C_{20}$ comportant une insaturation éthylénique non située en alpha de la fonction alcool.
- Les alcools secondaires ou tertiaires de formule R'-OH ou R' est un groupe alkyle linéaire ou ramifié de $C_3$ à $C_{20}$ ou un groupe cycloalkyle ou polycycloalkyle de $C_3$ à $C_{20}$ ou un groupe alkyle linéaire ou ramifié de $C_4$ à $C_{20}$ comportant une insaturation éthylénique non située en alpha de la fonction alcool.
- Les diols, les triols et les polyols comme l'éthylène glycol, le glycérol, le mannitol ou le sorbitol.
- Les aminoalcools comme ceux de formule $HOCH_2—CH(CH_3)—NH—CH_3$

On appelle aldoses les monosaccharides qui renferment une fonction aldéhyde comme les aldohexoses, les aldopentoses, les aldotétroses.

On appelle aldosides, les composés qui résultent de l'acétalisation d'un aldose avec un alcool soit par le procédé selon l'invention, soit par un autre procédé connu, par exemple un de ceux cités dans l'art antérieur. La réaction s'effectue alors par échange des groupements de substitution.

On appelle alkyl-disaccharides (méthyl-..., éthyl-..., etc...) les composés oligosaccharidiques qui résultent de la glycosidation par l'alcool présent dans le milieu réactionnel de di- ou oligo-saccharides susceptibles de résulter de la réaction, et principalement de disaccharides liés (1 —> 6) comme cela est illustré dans le schéma page 4.

On appelle polyaldosides, les oligo- et poly-saccharides qui résultent de la polymérisation des aldoses énumérés ci-dessus, y compris les disaccharides et les trisaccharides. Les polyaldosides sont de préférence choisis parmi les polysaccharides végétaux. L'amidon et la cellulose sont les polymères préférés. Les polysaccharides d'origine animale ou fongique (chitine, chitosane, etc...) conviennent également.

Parmi les aldoses, le glucose est le monosaccharide préféré.

Pour que la réaction s'effectue normalement, une condition supplémentaire doit être respectée, en particulier dans le cas où l'on met à réagir des polyaldosides : lors de la réaction entre l'aldose, l'aldoside, ou le polyaldoside et le fluorure d'hydrogène, des intermédiaires oligosaccharidiques sont susceptibles d'être formés. Si la quantité d'alcool est importante une forte proportion d'oligosaccharides présents dans le milieu aura tendance à précipiter ce qui déplacera l'équilibre du mélange en solution vers la formation d'oligomères. Ce phénomène a tendance à ralentir la vitesse de la réaction car il faut un délai supplémentaire pour que les oligomères soient resolubilisés. Ainsi, bien que la réaction soit effectuée de préférence en excès d'alcool par rapport au nombre d'équivalents monosaccharidiques présents il est souhaitable de ne pas dépasser la proportion (en mole) alcool : équivalent monosaccharidique égale à 60.

On appelle équivalents monosaccharidiques les composés susceptibles de résulter de l'hydrolyse totale des polyaldosides mis en réaction.

Par ailleurs, la quantité de fluorure d'hydrogène mis en œuvre dans la réaction conditionne la stabilité du fluorure de glucopyranosyle formé dans le milieu. En effet pour un rapport HF/équivalent monosaccharidique de 90 la quantité de fluorure de glucopyranosyle présent dans les produits finaux est supérieure à 50 % malgré un large excès d'alcool mis en jeu, d'autre part la dissolution et la fluorolyse de l'aldose, l'aldoside ou du polyaldoside nécessitent une quantité minimum d'HF ; on utilisera donc de préférence une proportion HF/équivalent monosaccharidique comprise entre 10 et 60 et mieux, entre 30 et 50.

Enfin, on a constaté qu'il était préférable d'utiliser des alcools primaires et de préférence des alcools aliphatiques comportant au plus 4 atomes de carbone. Il semble en effet que pour les alcools supérieurs la protonation de la fonction alcool puisse gêner le déroulement de la réaction.

Les alcools préférés sont choisis parmi le méthanol, l'éthanol, le propanol-1, le butanol-1, l'alcool allylique, le butène-3-ol-1.

Une première variante consiste à dissoudre l'aldose, l'aldoside, ou le polyaldoside dans le fluorure d'hydrogène de manière à provoquer la formation des intermédiaires réactifs précédemment mentionnés. Ensuite on ajoute au mélange l'alcool en excès de préférence dans une proportion supérieure à 20 fois et de préférence inférieure à 50 fois la quantité en mole d'aldoses, d'aldosides ou d'intermédiaires monosaccharidiques. La réaction est très rapide et conduit à la formation de l'alkyle ou cycloalkyle ou alcényle d'aldoside ou polyaldoside souhaité.

Toutefois, ce procédé nécessite l'emploi d'une quantité importante d'alcool. C'est pour cette raison que l'on préfère la variante suivante qui offre en plus l'avantage de permettre, problème crucial, la récupération de HF et, en outre, une amélioration du rendement.

A un mélange de HF et d'alcool, on ajoute, de préférence sous agitation, un aldose, un aldoside, ou polyaldoside, on sépare ensuite les composés résultants. De préférence, on ajoutera l'aldose, l'aldoside, ou le polyaldoside de telle façon qu'on obtienne une proportion d'alcool : équivalents monosaccharidiques comprise entre 3 et 20 en mole et mieux entre 6 et 15.

Lorsqu'on utilise un alcool plus lourd, à partir de $C_5$, un cosolvant facilitant la dissolution de ce réactif dans HF peut être nécessaire ; le dioxanne ou dioxyde de soufre sont des cosolvants appropriés dans ce cas. Ces solvants limitent par ailleurs la protonation de l'alcool.

Les exemples ci-dessous illustrent l'invention.

L'analyse des mélanges de méthyl-glucosides formés a été réalisée en r.m.n. du $^{13}C$ et chromatographie en phase gazeuse de dérivés acétylés et silylés selon l'une des méthodologies suivantes :

- Acétylation par l'anhydride acétique en présence de pyridine
- Silylation par le mélange de bis-triméthylsilyltrifluoroacétamide (BSTFA) et de triméthylchlorosilane (TMCS)
- Analyse sur colonne capillaire OV-1.

La teneur en eau des amidons utilisés a été mesurée par balance thermogravimétrique. Dans les exemples qui suivent, elle était, sauf indication contraire de :

- 10 % pour les amidons de blé et de maïs
- 14 % pour l'amidon de pomme de terre.

On notera que les rendements mentionnés dans les exemples ne sont pas optimisés, il doivent être considérés comme des valeurs minimales étant donné les conditions opératoires.

## Exemple 1

Synthèse de méthyl-D-glucopyranosides par dissolution préalable de l'amidon.

Dans un récipient en polyéthylène, 10 g d'amidon de maïs (0,055 mole) sont dissous dans 25 ml de fluorure d'hydrogène (1,25 mole) à 25 °C. Au bout de 15 minutes, sous agitation magnétique, 50 ml de méthanol (1,25 mole) sont ajoutés à la solution, soit 23 équivalents monosaccharidiques, et le mélange réactionnel est agité pendant 45 minutes. Le temps de réaction global est de 1 heure. Le mélange réactionnel est ensuite précipité dans 300 ml d'éther éthylique sous légère agitation magnétique, puis filtré. Le précipité est enfin séché sous pression réduite en présence de carbonate de calcium.

Le rendement de la réaction par rapport à la quantité d'amidon anhydre mis en œuvre est de 90 %. On obtient 9,7 g de produits dans les proportions suivantes :

| | |
|---|---|
| méthyl-D-glucopyranosides (alpha et bêta) | 50 % |
| méthyl-disaccharides (alpha et bêta) | 50 % |

Un essai effectué dans les mêmes conditions avec 40 ml de fluorure d'hydrogène (2 moles) et 100 ml de méthanol (2,5 moles) soit 45 équivalents monosaccharidiques conduit, avec un rendement de 95 %, à 10,2 g de produits en proportion suivante :

| | |
|---|---|
| méthyl-D-glucopyranosides (alpha et bêta) | 80 % |
| méthyl-disaccharides | 20 % |

## Exemple 2

Dans cet exemple ainsi que dans tous les exemples suivants on réalise la synthèse de l'alkyl-glucoside au moyen du mélange fluorure d'hydrogène-alcool.

A un mélange de fluorure d'hydrogène (2,5 moles) et de méthanol (0,75 mole) contenu dans un flacon en polyéthylène, on ajoute 10 g d'amidon de maïs à température ambiante. La quantité d'alcool est donc égale à 12,5 équivalents par équivalent monosaccharidique. Après la dissolution rapide de l'amidon le mélange est laissé sous agitation magnétique.

L'addition de 500 ml d'éther éthylique conduit à la précipitation des produits de réaction formés, qui sont séparés par filtration et lavés à l'éther. Leur séchage est effectué sous pression réduite en présence de carbonate de calcium et conduit à 9,2 g d'un composé sirupeux de couleur beige clair dont l'analyse montre qu'il est composé de :

| | |
|---|---|
| méthyl-alpha-D-glucopyranoside | 80 % |
| méthyl-bêta-D-glucopyranoside | 10 % |
| méthyl-disaccharides | 10 % |

Le rendement pondéral en méthyl-glucosides est de 85 % par rapport à l'amidon initial sec et on note que dans ces conditions, on n'observe la présence ni de glucose ni de dérivés glucofuranosidiques.

## Exemple 3

En reprenant les mêmes conditions opératoires que dans l'exemple 2, mais en divisant par deux la quantité de méthanol, on obtient une quantité plus importante de méthyl-disaccharides (20 %).

Après traitement, l'analyse du mélange de méthyl glucosides donne les résultats suivants :

| | |
|---|---|
| produits obtenus 9,5 (rendement 88 %) | |
| méthyl-alpha-D-glucopyranoside | 70 % |
| méthyl-bêta-D-glucopyranoside | 10 % |
| méthyl-disaccharides | 20 % |

Ce résultat fait apparaître que le fluorure de glucopyranoside réagit de façon similaire avec le méthanol et les hydroxyles primaires des unités glucose et que le rendement en monosaccharide est une fonction croissante du rapport molaire alcool/équivalents monosaccharidiques.

Exemple 4

Dans cet exemple et les suivants les essais on été conduits de façon à récupérer le mélange fluorure d'hydrogène-alcool résiduel. Ils ont été réalisés dans un réacteur en acier inoxydable (316 Ti), pouvant travailler jusqu'à 18 bars de pression, avec agitation magnétique. Le chauffage et le refroidissement sont assurés par une enveloppe double. Le réacteur, aux fins d'évaporation du mélange HF-alcool à la fin de la réaction, peut être mis en communication avec une pompe à palettes à double étage, donnant un vide moyen de 0,1 mm de mercure, le mélange est alors récupéré dans un condenseur à azote liquide. Cette indication du vide (0,1 mm de mercure) n'est nullement limitative.

10 g d'amidon de maïs (0,055 mole) sont mis en réaction dans un mélange de 50 ml de fluorure d'hydrogène (2,5 moles) et de 30 ml de méthanol (0,75 mole). La réaction est effectuée en 10 minutes à 40 °C. Puis la température est abaissée à 30 °C avant évaporation. L'excès de mélange HF-méthanol est évaporé sous pression réduite (0,1 mm Hg) en 20 minutes. 9,5 g d'un composé sirupeux rose-rouge sont récupérés présentant encore une légère acidité (0,1 % par rapport à HF mis en œuvre). Le mélange est repris par 20 ml d'eau et neutralisé par du carbonate de calcium. L'excès de carbonate de calcium ainsi que le fluorure de calcium formé sont filtrés et lavés à l'eau froide. Le filtrat est ensuite déminéralisé par une résine échangeuse d'ions mixte puis concentré à chaud sous pression réduite.

L'analyse du mélange incolore, finalement obtenu, sous forme de poudre donne les résultats suivants :

| | |
|---|---|
| Produits récupérés : 9,2 g | |
| Méthyl-alpha-D-glucopyranoside | 65 % |
| Méthyl-bêta-D-glucopyranoside | 25 % |
| Méthyl-disaccharides | 10 % |

Cet essai montre qu'une élévation de température accroît sensiblement la vitesse de réaction. Le taux de disaccharides reste constant, le chauffage n'influant que sur le rapport des anomères alpha/bêta du méthyl-glucoside.

Exemple 5

Des essais ont été conduits pour étudier l'influence de l'eau apportée par exemple par l'amidon lors des recyclages du mélange HF-alcool. Les essais ont été effectués sur l'amidon de blé non séché, à partir d'un mélange HF-méthanol bien défini en ajoutant directement des quantités croissantes d'eau dans HF.

A température ambiante, la vitesse de dissolution de l'amidon reste très rapide jusqu'à 0,4 équivalent $H_2O/HF$. Au-delà, la dissolution est ralentie.

Dans les conditions d'essai de l'exemple 4, 10 g d'amidon de blé sont dissous en 10 minutes à température ambiante dans un mélange de 50 ml de HF, 30 ml de méthanol et 2,5 ml d'eau soit 5 % par rapport à HF. Après 2 heures sous agitation, le mélange HF-alcool est évaporé. Le mélange sirupeux obtenu montre la présence de méthyl-D-glucopyranosides en mélange avec un reste d'oligosaccharides de l'amidon (5 %). Dans les mêmes conditions, un chauffage de 30 minutes à 40 °C, permet la fluorolyse et la méthanolyse complètes de l'amidon.

L'analyse du produit incolore obtenu a donné les résultats suivants :

| | |
|---|---|
| Masse produit brut : 9 g | |
| Méthyl-alpha-D-glucopyranoside | 60 % |
| Méthyl-bêta-D-glucopyranoside | 25 % |
| Méthyl-disaccharides | 14 % |
| Glucose | 1 % |

Par ailleurs, on a pu obtenir des résultats comparables dans ces mêmes conditions jusqu'à des concentrations en eau de 10 % par rapport à HF mis en jeu (équivalent à 5 à 6 recyclages du mélange si l'on part d'un amidon à 10 % d'humidité), ou jusqu'à des concentrations en eau de 15 % par rapport à HF mis en jeu.

Dans les conditions d'évaporation utilisées, le mélange de produits bruts obtenus retient de 40 à 50 % de l'humidité apportée par l'amidon représentant 5 % de la masse totale des produits formés.

Exemple 6

Le séchage préalable de l'amidon utilisé permettrait une récupération plus facile et plus efficace des mélanges HF-alcool après réaction.

Dans les conditions de l'exemple 4, 10 g d'amidon de blé séché, à 3 % de teneur en eau (séchage pendant 12 h à 50 °C sous 50 mm Hg) sont dissous en 5 minutes à température ambiante dans un mélange de 50 ml de méthanol.

Après 1 heure de réaction, le mélange HF-méthanol est évaporé. 10 g d'un composé sirupeux, pratiquement sec, sont récupérés, présentant encore une légère acidité (0,1 %/HF mis en jeu). L'analyse du produit donne les résultats suivants :

| | |
|---|---|
| Méthyl-alpha-D-glucopyranoside | 80 % |
| Méthyl-bêta-D-glucopyranoside | 10 % |
| Méthyl-disaccharides | 10 % |

La reprise par du méthanol chaud (20 ml) du mélange total puis refroidissement permet la cristallisation sélective du méthyl-alpha-D-glucopyranoside.

Dans cet exemple, le rendement en méthyl-alpha-D-glucopyranoside (contenant 1 à 2 % de méthyl-bêta-D-glucopyranoside) est de 60 % après 2 cristallisations successives.

Exemple 7

Afin de diminuer les quantités de réactif (HF, alcool) par rapport aux équivalents monosaccharidiques et limiter les quantités d'HF et d'alcool à évaporer, on constate que la diminution du rapport alcool/équivalent monosaccharidique conduit à une plus forte proportion en méthyl-disaccharides, comme le montre le tableau ci-dessous.

| | | | | | |
|---|---|---|---|---|---|
| Amidon de blé en g | 10 | 20 | 20 | 30 | 40 |
| Teneur en eau % | 10 | 10 | 3 | 10 | 10 |
| HF, ml | 50 | 50 | 50 | 50 | 50 |
| Méthanol, ml | 30 | 30 | 30 | 30 | 30 |
| Méthanol/amidon (mole/mole) | 13,6 | 6,8 | 6,8 | 4,5 | 3,4 |
| temps de réaction, h | 1 | 1 | 1 | 1 | 1 |
| T en °C | 40 | 40 | 40 | 40 | 40 |
| Méthyl-glucosides % | 90 | 85 | 85 | 75 | 65 |
| Méthyl-disaccharides % | 10 | 15 | 15 | 25 | 35 |

Ces essais ont été menés à 40 °C, car à une température inférieure on constate une réaction incomplète, avec présence de l'oligosaccharide non réagi, les conditions du tableau à partir de la deuxième colonne étant par ailleurs identiques. En effet une augmentation de température permet d'accélérer la vitesse de réaction quand on augmente la quantité d'amidon.

Exemple 8

On peut dans les conditions similaires à la synthèse de méthyl-glucosides obtenir les glucosides d'alcools supérieurs, jusqu'à $C_4$.

10 g d'amidon de blé (0,055 mole) sont dissous à température ambiante dans un mélange de 50 ml d'HF et de 44 ml d'éthanol absolu (0,75 mole) soit 13,6 équivalents par équivalent monosaccharidique. La réaction est conduite en 1 h 30 à température ambiante. Le mélange HF-éthanol est ensuite évaporé en 40 minutes sous pression réduite (0,1 mm Hg) à 25 °C. Le résidu est repris par le minimum d'eau (20 ml) et neutralisé par le carbonate de calcium. Après filtration, le filtrat est passé sur résine échangeuse d'ions mixte et concentré à sec.

L'analyse du produit obtenu a donné les résultats suivants :

| | |
|---|---|
| Masse produit brut : 9,0 g | |
| Ethyl-alpha-D-glucopyranoside | 75 % |
| Ethyl-bêta-D-glucopyranoside | 10 % |
| Ethyl-disaccharides | 15 % |

Exemple 9

20 g d'amidon de blé sont dissous à température ambiante dans un mélange de 50 ml d'HF et de 56 ml de propanol-1 (6,8 équivalents par équivalent monosaccharidique).

La réaction est conduite en 1 h à 40 °C sous agitation magnétique. Le mélange HF-propanol-1 est évaporé en 30 minutes à 40 °C sous 0,1 mm de Hg.

Après traitement comme dans l'exemple 8, l'analyse du mélange a donné les résultats suivants :

| | |
|---|---|
| Produits obtenus : 18,1 g | |
| Propyl-alpha-D-glucopyranoside | 65 % |
| Propyl-bêta-D-glucopyranoside | 15 % |
| Propyl-disaccharides | 20 % |

Exemple 10

10 g d'amidon de blé sont dissous dans un mélange de 50 ml d'HF (2,5 moles) et de 7 g d'éthylène glycol (0,112 mole, soit 2 équivalents moléculaires par équivalent monosaccharidique) à température ambiante, dans le réacteur. Après deux heures de réaction à température ambiante, HF est évaporé sous pression réduite en 20 minutes (sans entraînement de glycol). Le résidu est repris par 30 ml d'eau, neutralisé par le carbonate de calcium et filtré.

Le filtrat est concentré par évaporation. L'éthylène glycol résiduel est distillé par entraînement azéotropique avec le toluène.

L'analyse du mélange récupéré conduit aux résultats suivants :

| | |
|---|---|
| Masse produit brut : 9,4 g | |
| Glycol-mono-glucosides (alpha et bêta) | 70 % |
| Glycol-bis-glucosides | 20 % |
| Glycol-disaccharides | 10 % |

Dans les mêmes conditions, un excès d'éthylène glycol (0,22 mole-4 équivalents moléculaires) conduit au glycol-mono-glucoside avec un rendement de 85 %, pratiquement sans glycol-bis-glucoside ($< 5$ %).

Exemple 11

10 g d'amidon de blé sont dissous dans un mélange de 50 ml d'HF et de 16 ml de glycérol (0,22 mole) soit 4 équivalents moléculaires à température ambiante.

La réaction est laissée 2 h sous agitation magnétique à température ambiante.

HF est alors évaporé sous pression réduite en 30 minutes. Le mélange est repris par 50 ml d'eau, neutralisé par le carbonate de calcium et filtré ; le filtrat est déminéralisé sur résine mixte puis concentré.

L'analyse du produit obtenu conduit à détecter un mélange de composants comportant un rapport 7/3 de glycérol-glucosides et de glycérol-disaccharides ainsi que de glycérol non réagi.

Exemple 12

Cette technique de glycosidation directe des oses et polyosides dans HF donne des rendements convenables avec des alcools aliphatiques comportant au plus 4 atomes de carbone. Pour les alcools supérieurs, la protonation de la fonction hydroxyle, observée en r.m.n. du $^{13}C$, défavorise vraisemblable-

ment l'alkylation de l'ion glycosyl-oxycarbénium. Cet inconvénient peut être surmonté par dilution du fluorure d'hydrogène par le dioxanne ou le dioxyde de soufre. On a observé en effet, en prenant l'octanol-1 comme exemple, que l'espèce protonée de cet alcool diminue en fonction de la dilution du milieu par ces solvants. Ainsi, au-dessus d'une proportion 4/1 (v/v) de HF-dioxanne, l'octanol n'apparaît pas protoné. L'utilisation d'un mélange HF-dioxanne-octanol-1 a permis les synthèses d'octyl-glucosides illustrées dans les exemples suivants :

Préparation d'octyl-glucosides utilisant le mélange HF-dioxanne 4 : 1, (v/v).

A un mélange de fluorure d'hydrogène (25 ml, 1,25 mole) et de dioxanne (6,25 ml), on ajoute l'amidon (pomme de terre, teneur en eau ramenée à 10 %) 5 g, la solution est agitée pendant 15 minutes. L'octanol-1 (22 ml, 5 équivalents par équivalent monosaccharidique) est ajouté au mélange réactionnel puis la solution est conservée sous agitation pendant 30 minutes. Après évaporation des réactifs sous pression réduite (1 mm Hg), à température ambiante (récupération de HF-dioxanne) puis à 50° (récupération de l'octanol), 7,3 g d'un produit sirupeux sont obtenus. L'analyse montre qu'ils sont constitués de :

| | |
|---|---|
| Octyl-alpha-D-glucopyranoside | 55 % |
| Octyl-bêta-D-glucopyranoside | 5 % |
| Octyl-disaccharides | 25 % |
| Fluorure de glucopyranosyle | 15 % |

Le taux de conversion en octyl-glucosides, calculé par rapport à l'octanol est de 17 %.

Lorsque la proportion d'octanol par rapport aux équivalents monosaccharidiques présents est diminuée, la proportion de fluorure de glucopyranosyle augmente (cas de mélange réactionnel comportant de 1 à 2 équivalents d'octanol/équivalents monosaccharidiques). La proportion de ce fluorure de glucosyle peut être diminuée par dilution par le dioxanne. En effet, à partir d'un taux de dilution HF-dioxanne 2 : 1 (en volume), la quantité de fluorure de glucosyle présente n'est plus que de quelques pour cent. On recommande donc l'utilisation d'un mélange HF-dioxanne voisin de 2 : 1 (en volume) dans le cas où le nombre d'équivalents octanol par équivalent monosaccharidique est de 1 à 2, comme dans l'exemple ci-dessous.

## Exemple 13

Préparation d'octyl-glucosides utilisant un mélange HF-dioxanne 2 : 1 (en volume)

Les conditions opératoires sont identiques à celles de l'exemple précédent, mais les proportions sont les suivantes :

| | |
|---|---|
| Amidon (teneur en eau 10 %) | 5 g |
| HF | 25 ml |
| Dioxanne | 12,5 ml |
| Octanol-1 | 4,5 ml |

Après évaporation des réactifs, 7,2 g d'un produit sirupeux sont obtenus. L'analyse montre qu'il est constitué de :

| | |
|---|---|
| Octyl-glucosides | 35 % |
| Octyl-disaccharides | 40 % |
| Produits de réversion non glucosidés | 20 % |
| Fluorure de glucopyranosyle | 5 % |

Le taux de conversion par rapport à l'octanol mis en réaction est de 50 % environ.

## Exemple 14

Préparation de lauryl-glucosides utilisant un mélange HF-dioxanne, 2 : 1 (en volume)

A un mélange de fluorure d'hydrogène (50 ml, 2,5 moles) et de dioxanne (25 ml), on ajoute l'amidon (pomme de terre, teneur en eau 13 %, 10 g). La solution est agitée pendant 15 minutes. Le dodécanol-1 (12 ml, 1 équivalent par équivalent monosaccharide) est ajouté au mélange réactionnel, puis la solution est conservée sous agitation pendant 20 minutes à température ambiante. Le mélange est porté à 35 °C pendant 20 minutes pour l'évaporation du fluorure d'hydrogène à pression atmosphérique. L'évaporation de l'excès d'alcool ainsi que du dioxanne sous pression réduite (0,1 mm Hg) conduit à un produit solide (14,5 g). L'analyse montre qu'il est constitué des composants suivants :

| | |
|---|---|
| Lauryl-alpha-D-glucopyranoside | 25 % |
| Lauryl-bêta-D-glucopyranoside | 5 % |
| Lauryl-disaccharides | 10 % |
| Glucose / Produits de réversions | 55 % |
| Fluorure de glucopyranoside | < 5 % |

Le taux de conversion en Lauryl-glucoside par rapport à l'alcool laurique mis en œuvre est de 35 %.

**Revendications**

1. Procédé de synthèse d'aldosides ou d'oligo-aldosides d'alkyle, de cycloalkyle ou d'alcényle caractérisé en ce que l'on fait réagir un alcool aliphatique ou cycloaliphatique saturé ou un alcool aliphatique ou cycloaliphatique comportant une double liaison éthylénique non située en alpha du groupement hydroxyle avec un aldose, un aldoside ou un polyaldoside, dans un solvant et réactif constitué par du fluorure d'hydrogène.
2. Procédé de synthèse selon la revendication 1, caractérisé en ce que l'alcool est un alcool primaire.
3. Procédé de synthèse selon la revendication 2, caractérisé en ce que l'alcool est choisi parmi ceux comportant au plus quatre atomes de carbone.
4. Procédé de synthèse selon la revendication 1, caractérisé en ce que l'aldose est le glucose.
5. Procédé de synthèse selon la revendication 1, caractérisé en ce que le polyaldoside est choisi parmi les polysaccharides végétaux, amidon ou cellulose par exemple.
6. Procédé de synthèse selon la revendication 1, caractérisé en ce que le polyaldoside est choisi parmi les polysaccharides d'origine animale ou fongique.
7. Procédé de synthèse selon la revendication 1, caractérisé en ce que le rapport molaire fluorure d'hydrogène : équivalent monosaccharidique est compris entre 10 et 60.
8. Procédé de synthèse selon la revendication 1, caractérisé en ce que le rapport molaire alcool : équivalent monosaccharidique est inférieur ou égal à 60.
9. Procédé de synthèse selon la revendication 8, caractérisé en ce qu'on dissout l'aldose, l'aldoside ou le polyaldoside dans le fluorure d'hydrogène puis on ajoute l'alcool, le rapport molaire alcool : équivalent monosaccharidique est inférieur à 50.
10. Procédé de synthèse selon la revendication 8, caractérisé en ce qu'on ajoute à un mélange HF-alcool, un aldose, un aldoside, ou un polyaldoside, le rapport molaire alcool : équivalent monosaccharidique étant compris entre 3 et 20.
11. Procédé de synthèse selon la revendication 1, caractérisé en ce que la réaction est effectuée en milieu liquide.
12. Procédé de synthèse selon la revendication 1, caractérisé en ce que la quantité d'eau admise avec les réactifs reste de préférence inférieure à 15 % de la quantité de fluorure d'hydrogène.
13. Procédé de synthèse selon la revendication 1, caractérisé en ce que l'alcool est un diol, un triol ou polyol.
14. Procédé de synthèse selon la revendication 1, caractérisé en ce que l'alcool est un aminoalcool.
15. Procédé de synthèse selon la revendication 1, où l'alcool comporte au moins cinq atomes de carbone, caractérisé en ce qu'on ajoute un cosolvant approprié facilitant la dissolution de l'alcool dans le fluorure d'hydrogène et diminuant la force de protonation du milieu.
16. Procédé de synthèse selon la revendication 15, caractérisé en ce que le cosolvant est le dioxanne ou le dioxyde de soufre.

**Claims**

1. Process for the synthesis of alkyl, cycloalkyl or alkenyl aldosides or oligoaldosides, characterized in that a saturated aliphatic or cycloaliphatic alcohol or an aliphatic or cycloaliphatic alcohol containing an ethylenic double bond not situated in the alpha position with respect to the hydroxyl group is reacted with an aldose, an aldoside or a polyaldoside, in a solvent and reactant consisting of hydrogen fluoride.
2. Synthesis process according to Claim 1, characterized in that the alcohol is a primary alcohol.
3. Synthesis process according to Claim 2, characterized in that the alcohol is chosen from those containing not more than four carbon atoms.
4. Synthesis process according to Claim 1, characterized in that the aldose is glucose.
5. Synthesis process according to Claim 1, characterized in that the polyaldoside is chosen from plant polysaccharides, starch or cellulose, for example.
6. Synthesis process according to Claim 1, characterized in that the polyaldoside is chosen from polysaccharides of animal or fungal origin.
7. Synthesis process according to Claim 1, characterized in that the molar ratio hydrogen fluoride : monosaccharide equivalent is between 10 and 60.

8. Synthesis process according to Claim 1, characterized in that the molar ratio alcohol : monosaccharide equivalent is lower than or equal to 60.

9. Synthesis process according to Claim 8, characterized in that the aldose, aldoside or polyaldoside is dissolved in hydrogen fluoride and the alcohol is then added, the molar ratio alcohol : monosaccharide equivalent is lower than 50.

10. Synthesis process according to Claim 8, characterized in that an aldose, an aldoside or a polyaldoside is added to an HF-alcohol mixture, the molar ratio alcohol : monosaccharide equivalent being between 3 and 20.

11. Synthesis process according to Claim 1, characterized in that the reaction is performed in liquid medium.

12. Synthesis process according to Claim 1, characterized in that the quantity of water introduced with the reactants preferably remains below 15 % of the quantity of hydrogen fluoride.

13. Synthesis process according to Claim 1, characterized in that the alcohol is a diol, a triol or polyol.

14. Synthesis process according to Claim 1, characterized in that the alcohol is an aminoalcohol.

15. Synthesis process according to Claim 1, where the alcohol contains at least five carbon atoms, characterized in that a suitable cosolvent facilitating the dissolving of the alcohol in the hydrogen fluoride and reducing the protonation force of the medium is added.

16. Synthesis process according to Claim 15, characterized in that the cosolvent is dioxane or sulphur dioxide.

**Patentansprüche**

1. Verfahren zur Herstellung von Alkyl-, Cycloalkyl- oder Alkenyl-Aldosiden oder Oligoaldosiden, dadurch gekennzeichnet, daß man einen aliphatischen oder cycloaliphatischen gesättigten Alkohol oder einen aliphatischen bzw. cycloaliphatischen Alkohol mit einer ethylenartigen Doppelbindung, die nicht in α-Stellung zur Hydroxylgruppe ist, mit einer Aldose, einem Aldosid oder einem Polyaldosid in einem Lösungsmittel und Reagenz, bestehend aus Fluorwasserstoff, reagieren läßt.

2. Das Verfahren nach Anspruch 1, dadurch gekennzeichnet, daß es sich bei dem Alkohol um einen primären Alkohol handelt.

3. Das Verfahren nach Anspruch 2, dadurch gekennzeichnet, daß der Alkohol nicht mehr als höchstens 4 Kohlenstoffatome enthält.

4. Das Verfahren nach Anspruch 1, dadurch gekennzeichnet, daß es sich bei der Aldose um Glukose handelt.

5. Das Verfahren nach Anspruch 1, dadurch gekennzeichnet, daß das Polyaldosid aus der Gruppe der pflanzlichen Polysaccharide gewählt wird, z. B. Stärke oder Cellulose.

6. Das Verfahren nach Anspruch 1, dadurch gekennzeichnet, daß es sich bei dem Polyaldosid um ein tierisches Polysaccharid bzw. ein aus Pilzen stammendes handelt.

7. Das Verfahren nach Anspruch 1, dadurch gekennzeichnet, daß das molare Verhältnis zwischen Fluorwasserstoff und dem Monosaccharid-Äquivalent zwischen 10 und 60 liegt.

8. Das Verfahren nach Anspruch 1, dadurch gekennzeichnet, daß das molare Verhältnis zwischen Alkohol und dem Monosaccharid-Äquivalent gleich groß oder niedriger als 60 ist.

9. Das Verfahren nach Anspruch 8, dadurch gekennzeichnet, daß man die Aldose, das Aldosid oder das Polyaldosid in Fluorwasserstoff löst und daraufhin den Alkohol hinzufügt, wobei das molare Verhältnis Alkohol zu Monosaccharid-Äquivalent niedriger als 50 ist.

10. Das Verfahren nach Anspruch 8, dadurch gekennzeichnet, daß man zu einem HF-Alkoholgemisch eine Aldose, ein Aldosid oder ein Polyaldosid hinzufügt, wobei das molare Verhältnis Alkohol zu Monosaccharid-Äquivalent zwischen 3 und 20 liegt.

11. Das Verfahren nach Anspruch 1, dadurch gekennzeichnet, daß die Reaktion in flüssigem Medium ausgeführt wird.

12. Das Verfahren nach Anspruch 1, dadurch gekennzeichnet, daß die in Gegenwart der Reagenzien zulässige wassermenge vorzugsweise weniger als 15 % der Menge an Fluorwasserstoff beträgt.

13. Das Verfahren nach Anspruch 1, dadurch gekennzeichnet, daß es sich bei dem Alkohol um ein Diol, Triol oder Polyol handelt.

14. Das Verfahren nach Anspruch 1, dadurch gekennzeichnet, daß es sich bei dem Alkohol um einen Aminalkohol handelt.

15. Das Verfahren nach Anspruch 1, bei dem der Alkohol mindestens 5 Kohlenstoffatome enthält, dadurch gekennzeichnet, daß ein geeignetes Cosolvenz hinzugefügt wird, das die Lösung des Alkohols in dem Fluorwasserstoff erleichtert und die Protonierungskräfte des Mediums herabsetzt.

16. Das Verfahren nach Anspruch 15, dadurch gekennzeichnet, daß es sich bei dem Cosolvenz um Dioxan oder Schwefeldioxid handelt.